# EUROPÄISCHE PATENTANMELDUNG

(11) **EP 3 711 720 A1**
(43) Veröffentlichungstag der Anmeldung: **23.09.2020**
(21) Anmeldenummer: 19164035.8
(22) Anmeldetag: 20.03.2019
(51) Int. Cl.: A61F 13/06, A61F 5/01

(54) **VORRICHTUNG ZUR POSITIONERUNG UND/ODER SEPARIERUNG DER ZEHENGLIEDER EINES FÜSSES UND VERFAHREN ZUR HERSTELLUNG**

(71) Anmelder: Darco (Europe) GmbH, 82399 Raisting (DE)
(72) Erfinder: Dietrich, Thomas, 82399 Raisting (DE); Felix, Regina, 82399 Raisting (DE)
(74) Vertreter: Rupprecht, Kay

(57) **Zusammenfassung**

Die Erfindung betrifft eine Vorrichtung (1) zur Positionierung und/oder Separierung der Zehenglieder eines Fußes, mit einer Grundplatte (10), die ein Vorderende (10a) aufweist, ein Rückende (10b), sich einander gegenüberliegende erste und zweite Seitenenden (10c; 10d), eine Auftrittsfläche (11) und eine Grundfläche (12), und mit einer Mehrzahl von Trennstrukturen (20). Die Grundplatte (10) weist eine Querachse (X) von dem ersten Seitenende (10c) zu dem zweiten Seitenende (10d) auf und eine Längsachse (Y) von dem Vorderende (10a) zu dem Rückende (10b) der Grundplatte (10). Die Trennstrukturen (20) sind entlang der Auftrittsfläche (11) der Grundplatte (10) in Richtung der Querachse (X) voneinander beabstandet ausgebildet. Des Weiteren betrifft die Erfindung ein Verfahren zur Herstellung einer solchen Vorrichtung.

## Beschreibung

Die Erfindung betrifft eine Vorrichtung zur Positionierung und/oder Separierung der Zehenglieder eines Fußes und ein Verfahren zur Herstellung.

Um im Nachgang zu Operationen an einem Fuß, insbesondere minimalinvasiven Operationen, die erzielten Operationsergebnisse im Laufe der Wundheilung aufrechtzuerhalten und sicherzustellen, werden üblicherweise Bandagen angelegt, die der Fixation der einzelnen Zehenglieder dienen. Hierbei werden die einzelnen Zehen bzw. Zehenglieder eines Fußes derart bandagiert, dass unter anderem eine Separierung der Zehenglieder voneinander gewährleistet ist.

Derartige Bandagen erfordern einen großen Zeit-, Personal- sowie Materialaufwand und müssen regelmäßig erneuert werden, z.B. um dem Patienten die Körperhygiene oder die Wundpflege zu ermöglichen. Dabei erfordern Bandagierungen stets ein sorgfältiges Anlegen, um die einzelnen Zehenglieder korrekt zu positionieren und die Aufrechterhaltung der Operationsergebnisse während des Heilungsprozesses gewährleisten zu können.

Darüber hinaus ist zu beachten, dass sich jede neu angelegte Bandage in ihrer Ausgestaltung unterscheidet und somit eine Neupositionierung der Zehenglieder bedeutet. Eine Reproduzierbarkeit der ursprünglichen Ausrichtung der Zehenglieder ist bei einer neuen Bandagierung nicht gewährleistet.

Der Erfindung liegt die Aufgabe zu Grunde, eine Vorrichtung zur Positionierung und/oder Separierung der Zehen eines Fußes bereitzustellen, die einfach und kostengünstig herzustellen sowie an einem Patientenfuß anzulegen ist, eine sichere, reproduzierbare Positionierung und/oder Separierung der Zehen bereitstellt, sowie die Ausrichtung der Zehen in Extensionsstellung und die Plantarisierung der metatarsophalangealen Gelenke des Fußes auf einfache Weise ermöglicht.

Diese Aufgabe wird durch eine Vorrichtung nach Anspruch 1 sowie ein Verfahren nach Anspruch 13 gelöst. Bevorzugte Ausführungsformen sind jeweils in den abhängigen Ansprüchen angegeben.

Gemäß der vorliegenden Erfindung ist eine Vorrichtung zur Positionierung und/oder Separierung der Zehenglieder eines Fußes vorgesehen, mit einer Grundplatte, die ein Vorderende aufweist, ein Rückende, sich einander gegenüberliegende erste und zweite Seitenenden, eine Auftrittsfläche und eine Grundfläche, und mit einer Mehrzahl von Trennstrukturen. Die Grundplatte weist eine Querachse von dem ersten Seitenende zu dem zweiten Seitenende und eine Längsachse von dem Vorderende zu dem Rückende der Grundplatte auf. Die Trennstrukturen sind entlang der Auftrittsfläche der Grundplatte in Richtung der Querachse voneinander beabstandet ausgebildet.

Der Erfindung liegt die Idee zu Grunde, eine Vorrichtung bereitzustellen, die mittels der voneinander beabstandeten Trennstrukturen eine Positionierung und Separierung der Zehenglieder auf reproduzierbare Weise ermöglicht, insbesondere im Nachgang zu einer minimalinvasiven Operation am Fuß.

Hierzu wird die Vorrichtung mit Trennstrukturen bzw. Trennelementen bereitgestellt, die zur gezielten Positionierung und/oder Separierung der einzelnen Zehenglieder geeignet sind. So kann die Vorrichtung postoperativ wenigstens zeitweise angelegt und abgenommen werden, wobei eine gewünschte Anordnung der Zehen bzw. Zehenglieder anhand der Vorrichtung in reproduzierbarer Form bereitgestellt werden kann.

Insbesondere ist vorgesehen, dass die einzelne Trennstrukturen bzw. Trennelemente derart ausgestaltet sind, dass sie eine gezielte, dauerhafte Separierung und Positionierung der Zehenglieder untereinander ermöglichen. So können die Trennstrukturen als Trennwände ausgestaltet sein. Entlang der einzelnen Trennwände können sich die Zehenglieder jeweils einzeln abstützen.

Alternativ ist vorstellbar, dass die Trennstrukturen als Trennerhebungen der Auftrittsfläche ausgestaltet sind, sodass eine Separierung und Positionierung der Zehenglieder gewährleistet ist.

Gemäß einer bevorzugten Ausführungsform erstrecken sich die Trennstrukturen entlang der Auftrittsfläche in Richtung der Längsachse über wenigstens einen Teil der Grundplatte.

So können die einzelnen Zehenglieder im Rahmen des Heilungsprozesses nach einer Operation und im Sinne der Sicherstellung der Operationsergebnisse an dem Vorderende der Grundplatte zweckmäßig positioniert und separiert werden. Diese Ausrichtung bzw. Positionierung der Zehenglieder ist dabei auf einfache, zeitsparende und reproduzierbare Weise beim Anlegen der Vorrichtung an den Fuß eines Patienten bereitstellbar.

In einer Ausführungsform sind die Grundfläche und die Auftrittsfläche der Grundplatte über wenigstens einen Teil ihrer jeweiligen Erstreckung nicht parallel zueinander ausgebildet, sodass eine Plantarisierung der metatarsophalangealen Gelenke des Fußes bereitstellbar ist. Nach einer weiteren bevorzugten Ausführungsform weist die Auftrittsfläche über wenigstens einen Teil ihrer Erstreckung in Richtung der Längsachse eine Steigung gegenüber der Grundfläche auf, sodass die Grundplatte an dem Vorderende eine größere Dicke aufweist als an dem Rückende.

So kann anhand der Ausgestaltung bzw. Orientierung der Auftrittsfläche, im Vergleich zu der Grundplatte, eine vorteilhafte Ausrichtung der Zehenglieder in Extensionsstellung, also eine Streckung in Richtung des Fußrückens, und eine Plantarisierung der metatarsophalangealen Gelenke der Zehenglieder des Fußes bereitgestellt werden. Diese Ausrichtung ist beim Abnehmen und Anlegen der erfindungsgemäßen Vorrichtung auf vereinfachte Weise regelmäßig wiederherstellbar.

In einer bevorzugten Ausführungsform ist die Steigung der Auftrittsfläche derart gewählt, dass eine Plantarisierung der metatarsophalangealen Gelenke des Fußes erzielbar ist.

Bevorzugter Weise kann die über wenigstens einen Abschnitt der Auftrittsfläche vorgesehene Steigung bzw. Neigung konstant ausgebildet sein.

Somit ist anhand einer zumindest abschnittsweisen Neigung der Auftrittsfläche gegenüber der Grundfläche eine vorteilhafte, reproduzierbare Extensionsstellung der Zehenglieder sowie die Plantarisierung der metatarsophalangealen Zehengelenke gezielt sowie patientenindividuell sicherstellbar. Die mehrdimensionale, komplexe Ausrichtung der Zehenglieder zur Sicherung der Operationsergebnisse während des Heilungsprozesses kann auf einfache Weise sichergestellt werden.

Nach einer Ausführungsform ist die Steigung der Auftrittsfläche in Richtung der Längsachse der Grundplatte variabel ausgestaltet, insbesondere sodass die Auftrittsfläche in Richtung zu dem Vorderende eine zunehmende Steigung aufweist.

So kann die Auftrittsfläche zwischen dem Rückende und dem Vorderende eine beliebig bzw. progressiv zunehmende Steigung aufweisen, um eine zweckmäßige Extensionsstellung der Zehenglieder und die Plantarisierung der metatarsophalangealen Gelenke der Zehenglieder bereitzustellen.

Gemäß einer Ausführungsform ist die Grundplatte keilförmig ausgebildet.

Im Sinne einer keilförmigen Ausgestaltung der Grundplatte, kann die Auftrittsfläche über wenigstens einen Teil ihrer Erstreckung in Richtung der Längsachse der Vorrichtung eine Neigung bzw. Steigung aufweisen und über ihre restliche Erstreckung, insbesondere in einem Bereich des Vorderendes der Grundplatte, parallel zu der Grundfläche verlaufen.

Somit kann die Vorrichtung auf einfache und kostengünstige Weise herstellbar sein, wobei gleichzeitig die Orientierung der Zehenglieder in Extensionsstellung und die Plantarisierung der metatarsophalangealen Zehengelenke des Fußes sichergestellt werden kann.

In einer Ausführungsform ist der jeweilige Abstand zwischen den einzelnen Trennstrukturen von dem ersten Seitenende zu dem zweiten Seitenende hin, in Richtung der Querachse der Vorrichtung, kleiner werdend ausgestaltet oder die Trennstrukturen sind gleichmäßig voneinander beabstandet.

Anhand einer beliebigen Beabstandung der Trennstrukturen untereinander, kann die Vorrichtung an die patientenspezifische Anatomie adaptierbar sein. Vorzugsweise werden die Abstände zwischen den einzelnen Trennstrukturen bzw. Trennelementen zwischen dem ersten Seitenende und dem zweiten Seitenende kleiner, um eine geeignete Abstützung und Separierung der Zehenglieder verschiedener Größe bereitzustellen.

Nach einer weiteren bevorzugten Ausführungsform ist an einer Oberseite der Trennstrukturen wenigstens ein Dämpfungselement anordbar.

Mittels dem Dämpfungselement kann der Tragekomfort der erfindungsgemäßen Vorrichtung erhöht werden, wobei die Zehenglieder, insbesondere eine Fußrückenseite der Zehenglieder, mit dem Dämpfungselement in Kontakt kommen können. Ferner kann über das Dämpfungselement eine Kraft auf die Zehenglieder bzw. deren metatarsophalangealen Gelenke, wenigstens der äußeren vier Zehenglieder, zur zweckmäßigen Plantarisierung ausübbar sein.

Gemäß einer Ausführungsform ist das Dämpfungselement mit einer Fixationseinheit, insbesondere mit einem Fixationsband, entlang der Oberseite der Trennstrukturen anordbar, zur Befestigung der Vorrichtung an einem Fuß eines Patienten und/oder zur Ausübung eines Drucks auf den Fuß, sodass eine Plantarisierung der metatarsophalangealen Gelenke, insbesondere wenigstens eines Teils der Zehenglieder, des Fußes erzielbar ist.

So ist vorgesehen, dass das Dämpfungselement anhand einer Fixationseinheit entlang der Trennstrukturen anordbar und mit der Grundplatte verbindbar ist. Insbesondere kann das Dämpfungselement an der Oberseite der Trennstrukturen anordbar sein. Somit ist ein angenehmer Tragekomfort entlang der Zehenglieder beim Anlegen der erfindungsgemäßen Vorrichtung sichergestellt.

Des Weiteren ist mittels dem Dämpfungselement eine mechanische Kraft bzw. ein Druck entlang der Zehenglieder des Fußes derart ausübbar, dass die metatarsophalangealen Gelenke wenigstens eines Teils der Zehenglieder, insbesondere der äußeren vier Zehenglieder, zweckmäßig plantarisierbar sind.

In einer Ausführungsform ist die Fixationseinheit mit einem plattenförmigen Fixationselement in Kontakt mit dem Dämpfungselement ausgebildet.

Plattenförmig kann im Sinne der vorliegenden Erfindung als strukturelle Ausgestaltung des Fixationselementes der Fixationseinheit verstanden werden, oder als geometrische Ausgestaltung, wobei das Fixationselement eine weiche flexible Struktur aufweisen kann. Anhand des Fixationselementes kann eine großflächige Krafteinleitung auf das Dämpfungselement bereitgestellt werden, um eine geeignete und komfortable Befestigung der Vorrichtung an dem Fuß mittels der Fixationseinheit zu ermöglichen, sowie eine geeignete Kraftübertragung zur Erzielung der Plantarisierung der metatarsophalangealen Gelenke.

Alternativ können die Fixationseinheit, insbesondere das Fixationselement, und das Dämpfungselement einstückig ausgebildet sein.

Nach einer Ausführungsform ist das Fixationsband entlang einer Außenseite des Dämpfungselementes oder einer Außenseite des plattenförmigen Fixationselementes angeordnet, sowie entlang der Grundfläche der Grundplatte, zur Positionierung bzw. Befestigung der Vorrichtung an einem Fuß.

Insbesondere wird das Fixationsband um die Außenseite bzw. Außenseiten der Grundplatte und des Dämpfungselementes bzw. des Fixationselementes in Kontakt mit dem Dämpfungselement geführt, um eine zweckmäßige Fixierung der Vorrichtung an dem Fuß des Patienten zu ermöglichen. Das Fixationsband kann beispielsweise als Verschnürung für die Vorrichtung ausgebildet sein. Somit ist eine einfache und kostengünstige Anordnung und Befestigung der Vorrichtung bereitstellbar.

In einem nebengeordneten Aspekt der Erfindung ist ein Verfahren zur Herstellung einer erfindungsgemäßen Vorrichtung vorgesehen, wobei die Grundplatte, vorzugsweise in Kombination mit den Trennstrukturen, mittels eines Spritzgussverfahrens, mittels eines additiven Fertigungsverfahrens oder durch spanende Bearbeitung hergestellt wird.

Alternativ können die Trennstrukturen getrennt von der Grundplatte herstellbar sein, wobei die Trennstrukturen mit der Grundplatte verklemmbar sind, insbesondere entlang von zueinander korrespondierenden, miteinander verklemmbaren Geometrien. So können die Grundplatte und die Trennstrukturen modular ausgestaltbar und zweckmäßig miteinander verbindbar sein. Insbesondere ist eine patientenindividuelle Ausgestaltung der Trennstruktur verfügbar, wobei eine standardisierte Grundplatte, insbesondere eine Grundplatte für verschiedenste Fußgrößen, einsetzbar ist.

So kann die erfindungsgemäße Vorrichtung auf vielfältige, kostengünstige und patientenindividuelle Weise bereitstellbar sein. Des Weiteren können insbesondere die Grundplatte, die Trennstrukturen und das Fixationselement aus einem Metall, einem Kunststoff oder einem vergleichbaren, geeigneten Material ausgebildet sein.

Nach einer bevorzugten Ausführungsform wird die Grundplatte in Kombination mit den Trennstrukturen einstückig hergestellt. Auf diese Weise ist eine stabile Struktur der Vorrichtung bereitstellbar sowie eine einfache Anwendung sichergestellt ist.

Alternativ kann die erfindungsgemäße Vorrichtung, insbesondere die Grundplatte und die Trennstrukturen mehrstückig ausgebildet sein, wobei die Trennstrukturen im Sinne einer Klemmverbindung, einer Schnappverbindung oder dergleichen mit der Grundplatte verbindbar sein können.

Die Erfindung wird im Weiteren anhand der beigefügten Zeichnungen im Detail erläutert.

Es zeigen schematisch:
- Fig. 1: eine perspektivische Darstellung eines Ausführungsbeispiels einer Vorrichtung;
- Fig. 2: eine Seitendarstellung der Vorrichtung gemäß Fig. 1;
- Fig. 3: eine weitere perspektivische Darstellung der Vorrichtung gemäß Fig. 1; und
- Fig. 4: eine Draufsicht der Vorrichtung gemäß Fig. 1.

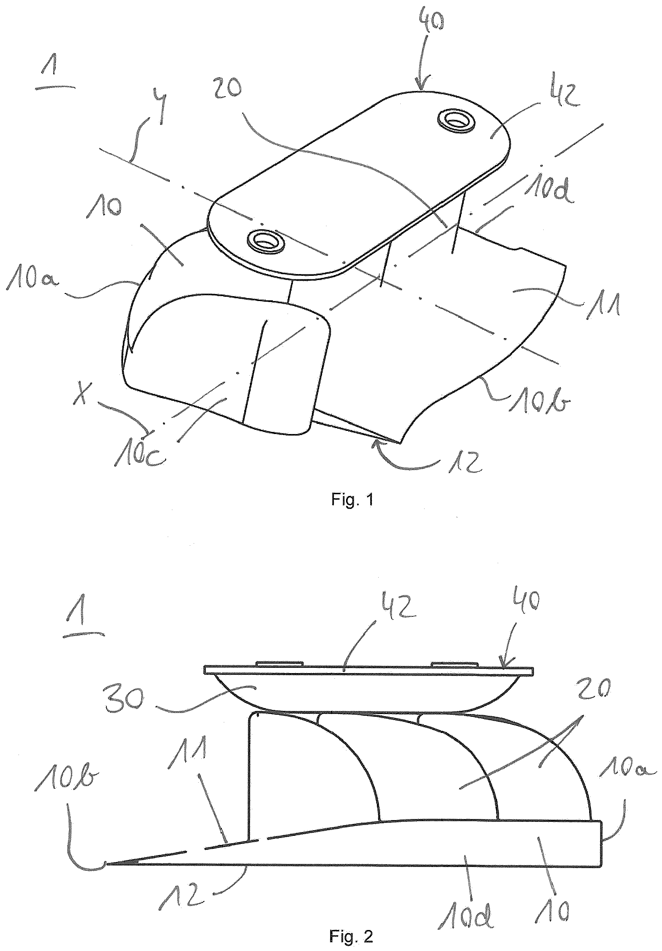

In Fig. 1 ist eine perspektivische Darstellung eines Ausführungsbeispiels einer Vorrichtung 1 gezeigt.

Die Vorrichtung 1 weist eine Grundplatte 10 mit einem Vorderende 10a, einem Rückende 10b, einem ersten Seitenende 10c und einem zweiten Seitenende 10d auf. Des Weiteren erstreckt sich die Grundplatte 10 entlang einer Längsachse Y zwischen dem Vorderende 10a und dem Rückende 10b sowie entlang einer Querachse X zwischen dem ersten Seitenende 10c und dem zweiten Seitenende 10d.

Das Vorderende 10a der Grundplatte 10 weist gemäß Fig. 1 einen bogenförmigen Verlauf auf. Das Rückenende 10b der Grundplatte 10 kann gemäß Fig. 1 einen wellenförmigen Verlauf aufweisen.

Die Grundplatte 10 weist eine Auftrittsfläche 11 und eine Grundfläche 12 auf. Die Auftrittsfläche 11 ist über wenigstens einen Teil der Grundplatte 10 bzw. der Auftrittsfläche 11 nicht parallel zu der Grundfläche 12 ausgebildet. Insbesondere ist die Auftrittsfläche 11 über wenigstens einen Teil ihrer Erstreckung in Richtung der Längsachse Y mit einer Neigung bzw. einer Steigung gegenüber der Grundfläche 12 ausgebildet. So weist die Grundplatte 10 an dem Vorderende 10a eine größere Dicke auf als an dem Rückende 10b. Insbesondere kann das Rückende 10b als ein spitz zulaufendes Ende zwischen der Auftrittsfläche 11 und der Grundfläche 12 ausgebildet sein, sodass eine auftretende Fußsohle zumindest im Wesentlichen nahtlos von einer Bodenfläche auf die Auftrittsfläche 11 übergehen kann.

Die Steigung der Auftrittsfläche ist gemäß Fig. 1 in Richtung der Längsachse Y über wenigstens einen Teil der Grundplatte 10 bzw. der Auftrittsfläche 11 konstant vorgesehen. So ist die Grundplatte 10 bevorzugter Weise keilförmig ausgebildet.

Alternativ kann die Grundplatte 10 in Richtung der Längsachse Y eine variable Steigung aufweisen, vorzugsweise eine von dem Rückende 10b zu dem Vorderende 10a zunehmende bzw. progressive Steigung.

Entlang der Grundplatte 10 sind in Richtung der Querachse X mehrere Trennstrukturen 20 angeordnet. Vorzugsweise sind insgesamt vier Trennstrukturen 20 vorgesehen, um die Zehenglieder eines Fußes zweckmäßig zu positionieren.

Die Trennstrukturen 20 erstrecken sich von dem Vorderende 10a der Grundplatte 10 im Wesentlichen in Richtung der Längsachse Y und in Richtung des Rückenendes 10b. Die Trennstrukturen 20 können unmittelbar an dem Vorderende 10a der Grundplatte 10 ansetzen oder im Bereich des Vorderendes gegenüber dem Vorderende 10a in Richtung der Längsachse Y zurückversetzt angeordnet sein.

Des Weiteren ist gemäß Fig. 1 vorgesehen, dass sich das erste Seitenende 10c im Sinne einer Seitenwand bzw. einer Trennstruktur-artigen Wand vertikal erstreckt.

Entlang einer Oberseite der Trennstrukturen 20 ist des Weiteren eine Fixationseinheit 40 gezeigt, insbesondere ein plattenförmiges Fixationselement 42 der Fixationseinheit 40. Das plattenförmige Fixationselement 42 kann sich im Wesentlichen in Richtung der Querachse X der Vorrichtung 1 erstrecken oder verdreht zu der Querachse X ausgerichtet sein.

Fig. 2 zeigt eine Seitendarstellung der Vorrichtung 1 gemäß Fig. 1.

Die Auftrittsfläche 11 der Grundplatte 10 weist abschnittsweise eine Steigung gegenüber der Grundfläche 12 auf. So ist die Grundplatte 10 im Sinne der vorliegenden Erfindung keilförmig ausgestaltet.

Die Trennstrukturen 20 sind zueinander in Richtung der Längsachse Y und der Querachse X versetzt zueinander vorgesehen. Die Trennstrukturen 20 sind im Bereich des Vorderendes 10a angeordnet.

Des Weiteren sind die Trennstrukturen 20 im Sinne eines bogenförmigen Kreisabschnitts ausgebildet und weisen eine bogenförmige Vorderseite bzw. Oberseite sowie eine vertikal verlaufende Rückseite auf.

Entlang der Oberseite der Trennstrukturen bzw. Trennelemente 20 ist ein Dämpfungselement 30 angeordnet. Das Dämpfungselement 30 kann als ein Dämpfungskissen oder dergleichen ausgebildet sein.

Entlang einer Oberseite des Dämpfungselementes 30 ist die Fixationseinheit 40 mit dem plattenförmigen Fixationselement 42 angeordnet. So kann mittels dem Fixationselement 42 sowohl das Dämpfungselement 30 positioniert als auch die Vorrichtung 1 an einem Fuß zweckmäßig angeordnet und befestigt werden.

Gemäß Fig. 1 und 2 weist das Fixationselement 42 hierzu in seinen seitlichen Endbereichen, in Richtung der Querachse X der Vorrichtung 1, Bohrungen auf, die beispielsweise zur Verklemmung, zur Verschraubung oder dergleichen mit der Grundplatte 10 eingesetzt werden können. So ist die Vorrichtung auf einfache modulare Weise an einem Fuß befestigbar.

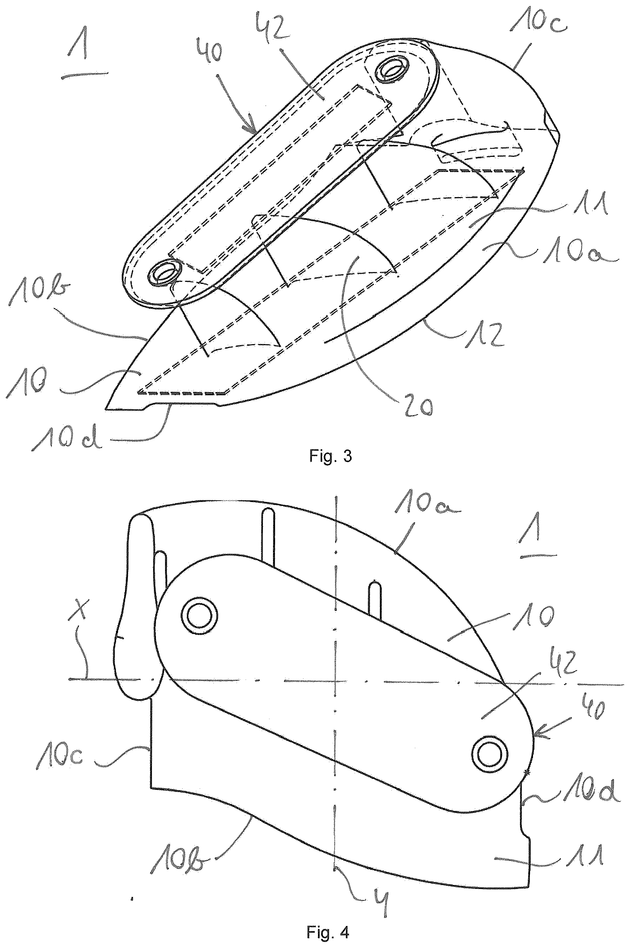

In Fig. 3 ist eine weitere perspektivische Darstellung der Vorrichtung 1 gemäß Fig. 1 gezeigt, insbesondere mit einer Veranschaulichung der verdeckten Kanten.

Gemäß Fig. 3 kann die Grundplatte einen rechteckigen Ausschnitt entlang der Auftrittsfläche 11 aufweisen. Die Trennstrukturen 20 können entlang eines dazu korrespondierenden Rechteckelementes ausgebildet sein, sodass die Trennstrukturen 20 entlang der Auftrittsfläche 11 mit der Grundplatte 10 verklemmbar sind.

Ebenso kann das Dämpfungselement 30 einen rechteckförmigen Ausschnitt aufweisen. Das Fixationselement 42 der Fixationseinheit 40 kann einen korrespondierenden, rechteckigen Vorsprung aufweisen, um eine zweckmäßige Verklemmung bzw. Ausrichtung des Dämpfungselementes 30 gegenüber dem Fixationselement 42 zu erzielen. Durch kraft- und/oder formschlüssige Verbindung des Fixationselementes 42 mit der Grundplatte 10 ist eine zweckmäßige Ausrichtung der Komponenten der Vorrichtung 1 gegenüber einem Fuß eines Patienten möglich.

In Fig. 4 ist eine Draufsicht der Vorrichtung 1 gemäß Fig. 1 gezeigt.

Die Grundplatte 10 ist mit einem bogenförmig ausgestalteten Vorderende 10a vorgesehen. Die Trennstrukturen 20 sind im Bereich des Vorderendes 10a in Richtung der Längsachse Y versetzt zueinander angeordnet.

Des Weiteren sind die Trennstrukturen 20 in Richtung der Querachse X versetzt zueinander angeordnet. Die Abstände zwischen den einzelnen Trennstrukturen 20 können in Richtung der Querachse X von dem ersten Seitenende 10c zu dem zweiten Seitenende 10d im Wesentlichen gleichmäßig ausgebildet sein. Alternativ können die Abstände zwischen den einzelnen Trennstrukturen 20 in Richtung der Querachse X von dem ersten Seitenende 10c zu dem zweiten Seitenende 10d kleiner werdend vorgesehen sein.

Das Fixationselement 42 der Fixationseinheit 40 erstreckt sich um ca. 20 Grad verdreht zu der Querachse X. Der Verlauf des Fixationselement 42 ist somit im Wesentlichen an dem bogenförmigen Vorderende 10a der Grundplatte 10 ausgerichtet.

An den seitlichen Enden des Fixationselementes 42 sind gemäß Fig. 4 Bohrungen vorgesehen, die zur Verschraubung, Verklemmung oder dergleichen mit der Grundplatte 10 einsetzbar sind. So ist eine zweckmäßige Befestigung der Vorrichtung 1 an dem Fuß eines Patienten verfügbar.

Zusammenfassend stellt die vorliegende Erfindung eine Vorrichtung bereit, die die vereinfachte und reproduzierbare Positionierung und/oder Separierung der einzelnen Zehenglieder eines Fußes, insbesondere im Nachgang zu einer minimalinvasiven Operation, ermöglicht.

Des Weiteren können die Zehenglieder anhand der erfindungsgemäßen Vorrichtung nicht nur positioniert bzw. separiert werden, sondern gleichzeitig entlang der metatarsophalangealen Gelenke der Zehenglieder des Fußes plantarisiert werden. Im Ergebnis sind anhand der vorgegebenen, reproduzierbaren Anordnung, Positionierung und Separation die erzielten Operationsergebnisse während der postoperativen Heilungsphase anhand der Vorrichtung auf einfache Weise sicherstellbar.

Des Weiteren stellt die Vorrichtung eine kostengünstige, zeitsparende, wiederverwendbare und einfach herzustellende Lösung zur zweckmäßigen Positionierung und/oder Separierung der einzelnen Zehenglieder eines Fußes dar.

### Bezugszeichenliste

- 1: Vorrichtung
- 10: Grundplatte
- 10a: Vorderende
- 10b: Rückende
- 10c: erstes Seitenende
- 10d: zweites Seitenende
- 11: Auftrittsfläche
- 12: Grundfläche
- 20: Trennstruktur
- 30: Dämpfungselement
- 40: Fixationseinheit
- 42: plattenförmiges Fixationselement

## Patentansprüche

1. Vorrichtung (1) zur Positionierung und/oder Separierung der Zehenglieder eines Fußes,
mit einer Grundplatte (10), die ein Vorderende (10a) aufweist, ein Rückende (10b), sich einander gegenüberliegende erste und zweite Seitenenden (10c; 10d), eine Auftrittsfläche (11) und eine Grundfläche (12), und mit einer Mehrzahl von Trennstrukturen (20),
wobei die Grundplatte (10) eine Querachse (X) von dem ersten Seitenende (10c) zu dem zweiten Seitenende (10d) aufweist und eine Längsachse (Y) von dem Vorderende (10a) zu dem Rückende (10b) der Grundplatte (10) aufweist,
wobei die Trennstrukturen (20) entlang der Auftrittsfläche (11) der Grundplatte (10) in Richtung der Querachse (X) voneinander beabstandet ausgebildet sind.

2. Vorrichtung (1) nach Anspruch 1,
**dadurch gekennzeichnet, dass**
sich die Trennstrukturen (20) entlang der Auftrittsfläche (11) in Richtung der Längsachse (Y) über wenigstens einen Teil der Grundplatte (10) erstrecken.

3. Vorrichtung (1) nach Anspruch 1 oder 2,
**dadurch gekennzeichnet, dass**
die Grundfläche (12) und die Auftrittsfläche (11) der Grundplatte (10) über wenigstens einen Teil ihrer jeweiligen Erstreckung nicht parallel zueinander ausgebildet sind, sodass eine Plantarisierung der metatarsophalangealen Gelenke des Fußes bereitstellbar ist.

4. Vorrichtung (1) nach einem der vorhergehenden Ansprüche,
**dadurch gekennzeichnet, dass**
die Auftrittsfläche (11) über wenigstens einen Teil ihrer Erstreckung in Richtung der Längsachse (Y) eine Steigung gegenüber der Grundfläche (12) aufweist, sodass die Grundplatte (10) an dem Vorderende (10a) eine größere Dicke aufweist als an dem Rückende (10b).

5. Vorrichtung (1) nach Anspruch 4,
**dadurch gekennzeichnet, dass**
die Steigung der Auftrittsfläche (11) derart gewählt ist, dass eine Plantarisierung der metatarsophalangealen Gelenke des Fußes erzielbar ist.

6. Vorrichtung (1) nach Anspruch 4 oder 5,
**dadurch gekennzeichnet, dass**
die Steigung der Auftrittsfläche (11) in Richtung der Längsachse (Y) der Grundplatte (10) variabel ausgestaltet ist, insbesondere sodass die Auftrittsfläche (11) in Richtung zu dem Vorderende (10a) eine zunehmende Steigung aufweist.

7. Vorrichtung (1) nach einem der Ansprüche 3 bis 6,
**dadurch gekennzeichnet, dass**
die Grundplatte (10) keilförmig ausgebildet ist.

8. Vorrichtung (1) nach einem der vorhergehenden Ansprüche,
**dadurch gekennzeichnet, dass**
der jeweilige Abstand zwischen den einzelnen Trennstrukturen (20) von dem ersten Seitenende (10c) zu dem zweiten Seitenende (10d) hin kleiner werdend ausgestaltet ist oder die Trennstrukturen (20) gleichmäßig voneinander beabstandet sind.

9. Vorrichtung (1) nach einem der vorhergehenden Ansprüche,
**dadurch gekennzeichnet, dass**
an einer Oberseite der Trennstrukturen (20) wenigstens ein Dämpfungselement (30) anordbar ist.

10. Vorrichtung (1) nach Anspruch 9,
**dadurch gekennzeichnet, dass**
das Dämpfungselement (30) mit einer Fixationseinheit (40), insbesondere mit einem Fixationsband, entlang der Oberseite der Trennstrukturen (20) anordbar ist, zur Befestigung der Vorrichtung 1 an einem Fuß eines Patienten und/oder zur Ausübung eines Drucks auf den Fuß, sodass eine Plantarisierung der metatarsophalangealen Gelenke des Fußes erzielbar ist.

11. Vorrichtung (1) nach Anspruch 10,
**dadurch gekennzeichnet, dass**
die Fixationseinheit (40) mit einem plattenförmigen Fixationselement (42) in Kontakt mit dem Dämpfungselement (30) ausgebildet ist.

12. Vorrichtung (1) nach Anspruch 9 oder 10,
**dadurch gekennzeichnet, dass**
das Fixationsband entlang einer Außenseite des Dämpfungselementes oder einer Außenseite des plattenförmigen Fixationselementes (42) angeordnet ist, sowie entlang der Grundfläche (12) der Grundplatte (10), zur Befestigung der Vorrichtung (1) an einem Fuß.

13. Verfahren zur Herstellung einer Vorrichtung (1) nach einem der vorhergehenden Ansprüche,
wobei die Grundplatte (10), vorzugsweise in Kombination mit den Trennstrukturen (20), mittels eines Spritzgussverfahrens, mittels eines additiven Fertigungsverfahrens oder durch spanende Bearbeitung hergestellt wird.

14. Verfahren nach Anspruch 13,
**dadurch gekennzeichnet, dass**
die Grundplatte (10) in Kombination mit den Trennstrukturen (20) einstückig hergestellt wird.
